Europäisches Patentamt

European Patent Office

Office européen des brevets

(19)

(11) Veröffentlichungsnummer: **0 176 957**
**B1**

(12) # EUROPÄISCHE PATENTSCHRIFT

(45) Veröffentlichungstag der Patentschrift:
19.04.89

(21) Anmeldenummer: 85112197.0

(22) Anmeldetag: 26.09.85

(51) Int. Cl.⁴: **A 61 B 17/32**

(54) **Chirurgisches Instrument.**

(30) Priorität: 02.10.84 CH 4722/84

(43) Veröffentlichungstag der Anmeldung:
09.04.86 Patentblatt 86/15

(45) Bekanntmachung des Hinweises auf die Patenterteilung:
19.04.89 Patentblatt 89/16

(84) Benannte Vertragsstaaten:
CH DE FR GB IT LI

(56) Entgegenhaltungen:
GB-A-2 044 103
US-A-2 944 552
US-A-4 290 427

(73) Patentinhaber: Levy, Ezra Dr., c/o Framarin via Butturini 9a, I - Verona (IT)

(72) Erfinder: Levy, Ezra Dr., c/o Framarin via Butturini 9a, I - Verona (IT)

(74) Vertreter: Hoeger, Stellrecht & Partner, Uhlandstrasse 14 c, D-7000 Stuttgart 1 (DE)

EP 0 176 957 B1

## Beschreibung

Die Erfindung betrifft ein chirurgisches Instrument zum Herausschneiden pathologischer Fisteln, insbesondere von Analfisteln, mit einem Griff und einem Schneidkopf.

Pathologische Fisteln sind bedauerlicherweise häufig. Sie werden durch einen Abszess, durch tuberkulöse Prozesse oder durch bösartige Tumoren oder dergleichen hervorgerufen. Analfisteln treten dabei weitaus am häufigsten auf; sie sind eine Folge von perianalen Abszessen oder von Hämorrhoiden. Analfisteln stellen eine Verbindung zwischen dem Mastdarm und der den Sitzmuskel überspannenden Haut dar, wobei der entstehende Kanal innerhalb oder außerhalb des Schließmuskels angeordnet sein kann. Analfisteln können nur auf chirurgischem Wege geheilt werden.

Um das infizierte Gewebe im Inneren einer Fistel herausschneiden zu können, ist man bei bekannten Operationsverfahren folgendermaßen vorgegangen: Man führt durch eine der beiden Öffnungen der Fistel einen Stab ein und schneidet mit einem Skalpell parallel zu diesem Stab, bis das Skalpell an der Wand der Fistel anlangt. Die Fistel ist dann über ihre gesamte Länge geöffnet. Das infizierte Gewebe wird herausgeschnitten und anschließend verbindet man, so gut dies möglich ist, die beiden Lippen des Einschnittes durch eine Naht oder durch Verklebung. Diese Verbindung ist entsprechend der Position der Fistel sehr schwer auszuführen. Insbesondere bei Analfisteln führt der Einschnitt auch zum Durchtrennen der Schleimhaut des Analkanals. Diese Schleimhaut kann nur sehr schwer vernäht werden. Bei innerhalb des Schließmuskels angeordneten Fisteln kann die Durchschneidung des Schließmuskels anschliessend eine chronische Inkontinenz erzeugen.

Es ist Aufgabe der vorliegenden Erfindung, ein neues chirurgisches Instrument vorzuschlagen, mit dem die Einschnitte in die Wand der Fistel vor dem Herausschneiden des kranken Gewebes vermieden werden, so daß man insbesondere bei Analfisteln einen Einschnitt der Schleimhaut des Analkanals und gegebenenfalls des Schließmuskels vermeiden kann.

Diese Aufgabe wird bei einem chirurgischen Instrument der eingangs beschriebenen Art erfindungsgemäß dadurch gelöst, daß der Griff mit einem Zugglied verbunden ist, daß der Schneidkopf eine ringförmige Klinge mit einer Schneide aufweist, an deren Innenwand sich mindestens zwei sich radial nach innen erstreckende Schneidklingen anschließen, die zu einer in der Mitte der ringförmigen Klinge angeordneten Verbindungsnabe führen, welche mit dem Zugglied verbindbar ist, und daß die Schneiden der ringförmigen Klinge und der radialen Schneidklingen auf der dem Griff zugewandten Seite angeordnet sind.

Der Vorteil dieses Instrumentes liegt darin, daß kein seitlicher Einschnitt mehr notwendig ist, um die Fistelwand zu erreichen und das kranke Gewebe herauszuschneiden. Mit dem beschriebenen Instrument wird folgendermaßen gearbeitet: Man führt das am Griff befestigte Zugglied durch die äußere Öffnung der Fistel ein und schiebt es vor, bis das Zugglied an der zweiten Öffnung der Fistel wieder austritt. Am freien Ende befestigt man die Verbindungsnabe der ringförmigen Klinge an dem Zugglied in der Weise, daß die Schneiden in Richtung auf die Fistel weisen. Daran anschließend zieht man an dem Zugglied in der umgekehrten Richtung, das heißt, die ringförmige Schneide wird durch die zweite Öffnung in die Fistel hineingezogen und tritt danach durch die erste Öffnung der Fistel wieder aus, wobei das von der ringförmigen Schneide umschlossene Gewebe herausgeschnitten wird. Dieses Herausschneiden kann mehrfach wiederholt werden, bis man sicher ist, daß das gesamte infizierte Gewebe herausgeschnitten ist. Selbstverständlich kann man dabei ringförmige Schneiden unterschiedlicher Durchmesser verwenden, die unterschiedlich großen Fisteln entsprechen.

Am Ende der Schneidoperation genügt es, die beiden Öffnungen der Fistel durch Vernähen zu schließen, eventuell genügt es sogar, nur die innere Öffnung zu verschließen.

Günstig ist es, wenn die Schneide der radialen Schneidklingen von der Verbindungsnabe zur ringförmigen Klinge hin geneigt ist, und zwar derart, daß die Schneidklinge in dem der Verbindungsnabe benachbarten Bereich dem Griff näher liegt als im radial außenliegenden Bereich. Dadurch ergibt sich beim Durchziehen des Schneidkopfes durch den Fistelkanal ein ziehender Schnitt für diese Schneiden, so daß der Schneidvorgang mit geringeren Verletzungen des umgebenden Gewebes erfolgen kann.

Bei einer Analfistel ist die äußere Öffnung, durch die man zunächst das Zugglied einführt, im Bereich der den Sitzmuskel überspannenden Haut angeordnet, während sich die innere oder zweite Öffnung im Mastdarm befindet. Da bei dieser Art des Vorgehens ein seitlicher Einschnitt in die Fistel nicht mehr notwendig ist, vermeidet man auch einen unnötigen Einschnitt der Schleimhaut und gegebenenfalls des Schließmuskels.

Bei einer bevorzugten Ausführungsform trägt das freie Ende des Zuggliedes ein Gewinde, welches in eine Gewindebohrung der Verbindungsnabe einschraubbar ist. Damit ist in einfacher Weise eine Verbindung zwischen Schneidkopf und Zugglied herstellbar.

Die Zahl der radialen Schneidklingen beträgt mindestens zwei, in einem bevorzugten Ausführungsbeispiel können drei radial verlaufende Schneidklingen vorgesehen sein, die gegeneinander jeweils um 120° versetzt sind.

Günstig ist es, wenn die Schneiden der radialen Schneidklingen nicht genau radial verlaufen, sondern geringförmig bogenförmig von innen nach außen verlaufen.

Eine besonders vorteilhafte Ausführungsform

ergibt sich, wenn die Schneidklingen in axialer Richtung des Schneidkopfes in der Art von Schaufeln schraubenförmig gebogen verlaufen. Wenn man den Schneidkopf durch den Fistelkanal hindurchzieht, wird dadurch der Schneidkopf um seine Längsachse gedreht, so daß auch im Bereich der ringförmigen Schneide ein ziehender Schnitt erfolgt. Die Vermeidung des reinen Druckschnittes führt zu geringeren Verletzungen des umliegenden Gewebes.

Das Zugglied kann ein Stab sein, vorzugsweise verwendet man als Zugglied ein flexibles Seil, wobei die Flexibilität so gering gehalten wird, daß das Zugglied in der beschriebenen Weise in den Fistelkanal eingeschoben werden kann. Vorzugsweise besteht das verwendete Seil aus einer Vielzahl von miteinander verdrehten Drähten, die dem Seil zwar eine gewisse Festigkeit verleihen, die aber auch eine bleibende Verbiegung des Seiles ermöglichen.

Die nachfolgende Beschreibung bevorzugter Ausführungsformen der Erfindung dient im Zusammenhang mit der Zeichnung der näheren Erläuterung.

Es zeigen:

Figur 1: eine Seitenansicht des Griffes und des Zuggliedes eine Fistelschneidinstrumentes;
Figur 2: eine Seitenansicht des Schneidkopfes;
Figur 3: eine Schnittansicht längs Linie III-III in Figur 4;
Figur 4: eine Draufsicht auf den Schneidkopf;
Figur 5: eine Ansicht ähnlich Figur 1 eines abgewandelten Ausführungsbeispiels eines Fistelschneidinstruments;
Figur 6: eine Ansicht ähnlich Figur 3 eines weiteren Ausführungsbeispieles eines Schneidkopfes und
Figur 7: eine Draufsicht auf den Schneidkopf der Figur 6.

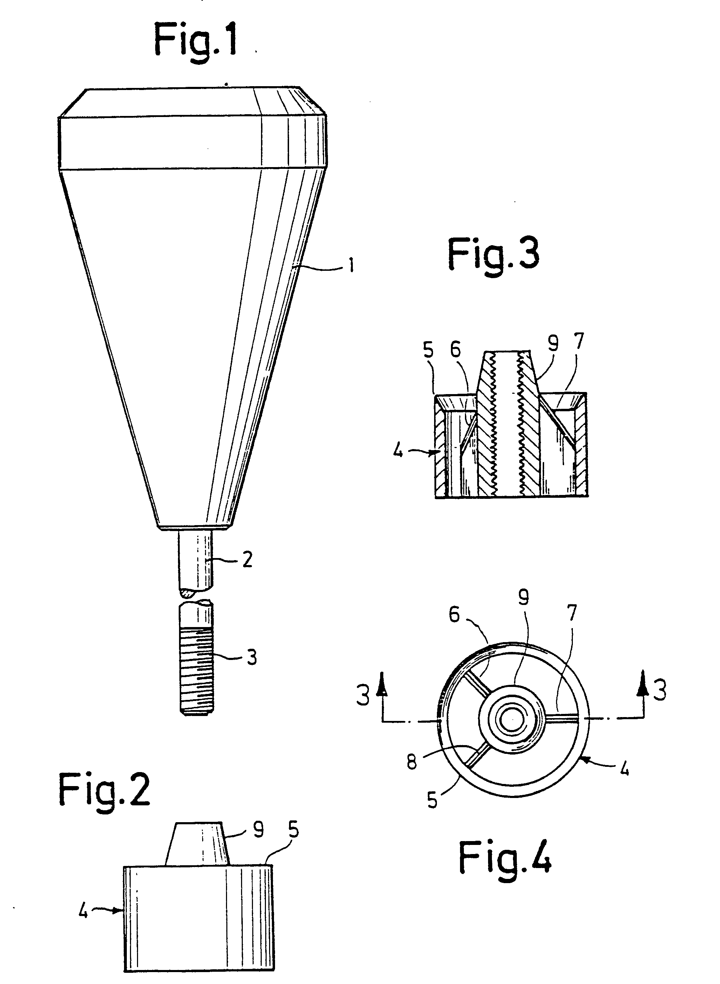

Das in den Figuren 1 bis 4 dargestellte Instrument umfaßt einen Handgriff 1, der an einem Ende einen Stab 2 trägt, dessen freies Ende 3 mit einem Außengewinde versehen ist. Ein Schneidkopf bildet den zweiten Teil des chirurgischen Instrumentes. Dieser umfaßt eine ringförmige Klinge 4, deren eine Umfangskante 5 in Form einer Schneide geschliffen ist. Weiterhin umfaßt der Schneidkopf drei ebene Schneidklingen 6, 7, 8, die sich von der ringförmigen Klinge 6 radial zur Mitte hin erstrecken. Die Schneidklingen 6, 7, 8 sind mit einer zentralen Verbindungsnabe 9 verbunden, die eine Gewindebohrung aufweist, in die das freie Ende 3 des Stabes 2 einschraubbar ist. Die Verbindungsnabe 9 ist mit den Innenkanten der Schneidklinge 6, 7, 8 verbunden, die gegenüberliegenden Außenkanten dieser Schneidklingen sind fest mit der Innenwand der ringförmigen Klinge 4 verbunden. Die Kanten der Schneidklinge 6, 7, 8, die auf derselben Seite liegen wie die Umfangsschneide 5 der Klinge 4, sind ebenfalls schneidenförmig geschliffen und sind vorzugsweise von der Verbindungsnabe 9 zur Umfangsschneide 5 der ringförmigen Klinge hin abfallend geneigt.

Die Länge des Stabes 2 kann beispielsweise 100 mm betragen, sein Durchmesser 1 mm, die Länge des Schneidkopfes 10 mm, sein Durchmesser etwa 5 mm. Selbstverständlich können die Gesamtabmessungen und auch die Abmessungen der Einzelteile des Instrumentes unterschiedlich gewählt und an die Größe der auszuschneidenden Fistel angepaßt werden.

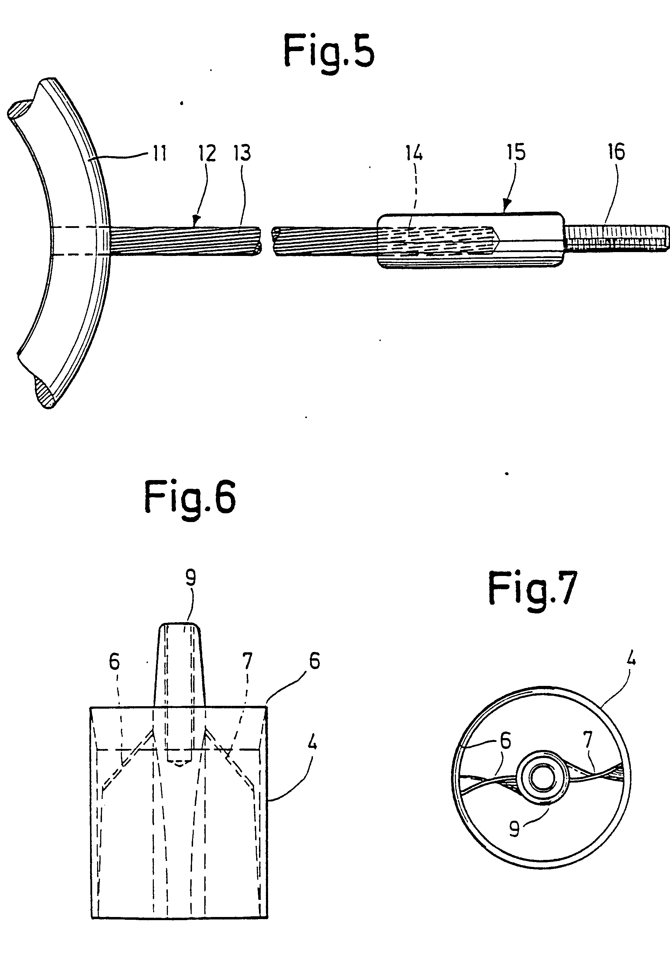

Bei dem in Figur 5 dargestellten Ausführungsbeispiel ist anstelle eines knopfförmigen Handgriffs 1 ein ringförmiger Handgriff 11 vorgesehen, an dem beispielsweise durch Hartlötung ein aus Einzeldrähten 13 aufgebautes polyfiles Seil 12 befestigt ist. Das freie Ende 14 des Seils 12 ist in ein Verbindungsstück 15 eingesetzt und mit diesem beispielsweise durch Hartlötung verbunden. Dieses Verbindungsstück zeigt einen Außengewindezapfen 16, der in die Gewindebohrung in der Verbindungsnabe 9 des Schneidkopfes einschraubbar ist.

In den Figuren 6 und 7 ist eine abgewandelte Ausführungsform eines Schneidkopfes dargestellt. Dieser ist weitgehend ähnlich aufgebaut wie der Schneidkopf der Figuren 2 bis 4, gleiche Teile tragen daher dieselben Bezugszeichen.

Der Schneidkopf der Figuren 6 und 7 weist lediglich zwei diametral einander gegenüberliegende Schneidklingen 6 und 7 auf. Diese sind im Gegensatz zu den Schneidklingen des Ausführungsbeispiels der Figuren 2 bis 4 nicht eben ausgebildet, sondern verlaufen von innen nach außen bogenförmig (Figur 7) und sind außerdem in Axialrichtung des Schneidkopfes in der Art von schraubenförmig gebogenen Schaufeln ausgebildet, wie dies ebenfalls aus der Zeichnung deutlich wird. Durch die schraubenförmige Ausgestaltung der schaufelförmigen Schneidklingen wird der gesamte Schneidkopf in eine Drehung um seine Längsachse versetzt, wenn der Schneidkopf durch das Gewebe gezogen wird. Dies führt dazu, daß die Umfangsschneide 5 mit dem Gewebe in eine ziehende Schneidverbindung gelangt, so daß der Schneidvorgang begünstigt wird.

**Patentansprüche**

1. Chirurgisches Instrument zum Herausschneiden pathologischer Fisteln mit einem Griff und einem Schneidkopf, dadurch gekennzeichnet, daß der Griff (1, 11) mit einem Zugglied (2, 12) verbunden ist, daß der Schneidkopf eine ringförmige Klinge (4) mit einer Schneide (5) aufweist, an deren Innenwand sich mindestens zwei sich radial nach innen erstreckende Schneidklingen (6, 7, 8) anschliessen, die zu einer in der Mitte der ringförmigen Klinge (4) angeordneten Verbindungsnabe (9) führen, welche mit dem Zugglied (2, 12) verbindbar ist, und daß die Schneiden (5) der ringförmigen Klinge (4) und der radialen Schneidklingen (6, 7, 8) auf der dem Griff (1, 11) zugewandten Seite angeordnet sind.

2. Instrument nach Anspruch 1, dadurch gekennzeichnet, daß das freie Ende (3, 14, 15, 16) des Zuggliedes (2, 12) ein Gewinde trägt, welches in eine Gewindebohrung der Verbindungsnabe (9) einschraubbar ist.

3. Instrument nach Anspruch 1 oder 2, dadurch gekennzeichnet, daß die Schneide der radialen Schneidklingen (6, 7, 8) von der Verbindungsnabe (9) zur ringförmigen Klinge (4) hin geneigt ist.

4. Instrument nach einem der voranstehenden Ansprüche, dadurch gekennzeichnet, daß drei radial verlaufende Schneidklingen (6, 7, 8) vorgesehen sind.

5. Instrument nach einem der Ansprüche 1 bis 4, dadurch gekennzeichnet, daß die Schneiden der radialen Schneidklingen (6, 7, 8) bogenförmig von innen nach außen verlaufen.

6. Instrument nach einem der Ansprüche 1 bis 5, dadurch gekennzeichnet, daß die Schneidklingen (6, 7, 8) in axialer Richtung des Schneidkopfes in der Art von Schaufeln schraubenförmig gebogen verlaufen.

7. Instrument nach einem der voranstehenden Ansprüche, dadurch gekennzeichnet, daß das Zugglied ein Stab (2) ist.

8. Instrument nach einem der Ansprüche 1 bis 6, dadurch gekennzeichnet, daß das Zugglied ein flexibles Seil (12) ist.

9. Instrument nach Anspruch 8, dadurch gekennzeichnet, daß das Seil (12) aus einer Vielzahl von miteinander verdrehten Drähten (13) besteht.

## Claims

1. Surgical instrument for cutting out pathological fistulae, having a handle and a cutting head, characterized in that the handle (1, 11) is connected to a tension member (2, 12), in that the cutting head has a ring-shaped blade (4) with a cutting edge (5) to the inside wall of which blade are connected at least two cutting blades (6, 7, 8) which extend radially inwards, these cutting blades going to a connecting hub (9) which is arranged in the middle of the ring-shaped blade (4) and can be connected to the tension member (2, 12), and in that the cutting edges (5) of the ring-shaped blade (4) and of the radial cutting blades (6, 7, 8) are arranged on the side facing the handle (1, 11).

2. Instrument according to Claim 1, characterized in that the free end (3, 14, 15, 16) of the tension member (2, 12) bears a thread which can be screwed into a tapped hole of the connecting hub (9).

3. Instrument according to Claims 1 or 2, characterized in that the cutting edge of the radial cutting blades (6, 7, 8) is inclined from the connecting hub (9) to the ring-shaped blade (4).

4. Instrument according to one of the preceding Claims, characterized in that three radial cutting blades (6, 7, 8) are provided.

5. Instrument according to one of Claims 1 to 4, characterized in that the cutting edges of the radial cutting blades (6, 7, 8) run from the inside to the outside in a curve.

6. Instrument according to one of Claims 1 to 5, characterized in that in the axial direction of the cutting head, the cutting blades (6, 7, 8) run spirally curved in the manner of vanes.

7. Instrument according to one of the preceding Claims, characterized in that the tension member is a bar (2).

8. Instrument according to one of Claims 1 to 6, characterized in that the tension member is a flexible rope (12).

9. Instrument according to Claim 8, characterized in that the rope (12) is composed of a multiplicity of wires (13) which are twisted together.

## Revendications

1. Instrument chirurgical pour ectomiser des fistules pathologiques, comportant une poignée et une tête de coupe, caractérisé en ce que la poignée (1, 11) est reliée à un organe de tirage (2, 12); en ce que la tête de coupe présente une lame annulaire (4) présentant un tranchant (5) à la paroi intérieure duquel se raccordent au moins deux lames de coupe (6, 7, 8) qui s'étendent radialement vers l'intérieur et rejoignent un moyeu de liaison (9) qui est disposé dans l'axe de la lame annulaire (4) et que l'on peut relier avec l'organe de tirage (2, 12); et en ce que les tranchants (5) de la lame annulaire (4) et des lames de coupe radiales (6, 7, 8) sont disposés du côté tourné vers la poignée (1, 11).

2. Instrument selon la revendication 1, caractérisé en ce que l'extrémité libre (3, 14, 15, 16) de l'organe de tirage (2, 12) porte un filetage qui peut se visser dans un taraudage du moyeu de liaison (9).

3. Instrument selon la revendication 1 ou 2, caractérisé en ce que le tranchant des lames de coupe radiales (6, 7, 8) est incliné en allant du moyeu de liaison (9) vers la lame annulaire (4).

4. Instrument selon l'une des revendications précédentes, caractérisé en ce qu'il est prévu trois lames de coupe (6, 7, 8) orientées radialement.

5. Instrument selon l'une des revendicatins 1 à 4, caractérisé en ce que les tranchants des lames de coupe radiales (6, 7, 8) sont en forme d'arc orienté de l'intérieur vers l'extérieur.

6. Instrument selon l'une des revendications 1 à 5, caractérisé en ce que les lames de coupe (6, 7, 8) sont, dans la direction axiale de la tête de coupe, inclinées en hélice à la façon de pales.

7. Instrument selon l'une des revendications précédentes, caractérisé en ce que l'organe de tirage est une tige (2).

8. Instrument selon l'une des revendications 1 à 6, caractérisé en ce que l'organe de tirage est un câble flexible (12).

9. Instrument selon la revendication 8, caractérisé en ce que le câble (12) est constitué d'une pluralité de fils (13) tordus les uns avec les autres.

# Fig.1

# Fig.3

# Fig.2

# Fig.4

# Fig.5

11 12 13      14 15 16

# Fig.6

9

6   7   6

4

# Fig.7

4

6   7

9